Europäisches Patentamt

**European Patent Office**

Office européen des brevets

(11) Publication number: **0 067 555**
**A2**

(12) # EUROPEAN PATENT APPLICATION

(21) Application number: **82302585.3**

(22) Date of filing: **20.05.82**

(51) Int. Cl.³: **A 61 B 1/00**

(30) Priority: **22.05.81 JP 77827/81**
**22.05.81 JP 77828/81**
**22.05.81 JP 77830/81**
**25.05.81 JP 78924/81**
**05.04.82 JP 55341/82**

(43) Date of publication of application:
**22.12.82 Bulletin 82/51**

(84) Designated Contracting States:
**BE CH DE FR GB IT LI**

(71) Applicant: **Fuji Photo Optical Co., Ltd.**
**1-324 Uetake-cho Omiya-shi**
**Saitama-ken(JP)**

(72) Inventor: **Ohshiro, Susumu**
c/o Fuji Photo Optical Co. Ltd. 1-324, Uetake-cho
Omiya-shi Saitama-ken(JP)

(72) Inventor: **Arakawa, Satoshi**
c/o Fuji Photo Optical Co. Ltd. 1-324, Uetake-cho
Omiya-shi Saitama-ken(JP)

(72) Inventor: **Kishi, Yukitoshi**
c/o Fuji Photo Optical Co. Ltd. 1-324, Uetake-cho
Omiya-shi Saitama-ken(JP)

(72) Inventor: **Kojima, Takuo**
c/o Fuji Photo Optical Co. Ltd. 1-324, Uetake-cho
Omiya-shi Saitama-ken(JP)

(72) Inventor: **Soriano, Leon**
23 Avenue Bon Air 1640 Rhode Street
Genese(BE)

(74) Representative: **Ayers, Martyn Lewis Stanley et al,**
J.A. KEMP & CO. 14 South Square Gray's Inn
London, WC1R 5EU(GB)

(54) **Apparatus for restoring the light transmittance of an image-transmitting optical fiber bundle used in fiberoptic endoscope.**

(57) When a fiberoptic endoscope is exposed to X-ray or γ-ray irradiation, the fiber bundle becomes discolored and there is thus a reduction in light transmittance. This makes observation or examination difficult or impossible. The irradiation-induced reduction in light transmittance of the image-transmitting fiber bundle is restored by visible light radiation. The image-transmitting fiber bundle at either end is exposed to visible light radiation which is conducted from a light source by a light transmitting means using an attachment which is fitted when restoration is required.

FIG. 3

EP 0 067 555 A2

Croydon Printing Company Ltd.

- 1 -

## DESCRIPTION

"APPARATUS FOR RESTORING THE LIGHT TRANSMITTANCE
OF AN IMAGE-TRANSMITTING OPTICAL FIBER BUNDLE
USED IN FIBEROPTIC ENDOSCOPE"

The present invention relates to an apparatus for restoring the reduced light transmittance of an image-transmitting optical fiber bundle which reduction result from X-ray or γ-ray irradiation, and for thus making it once more usable for performing observation or examinations.

Fiberoptic endoscopes in general use have two optical fiber bundles built therein, one for transmitting an image of the internal parts to be observed or examined and the other for transmitting illumination light from the outside. Each of said optical fiber bundles comprises an extremely large number of optical fibers with their opposed end portions rigidly secured together and they are free along their extent between the ends to be flexible so as to be insertable along a tortuous passage of a body. Fiberoptic endoscopes which are used to observe or examine inaccessible body cavities which are impossible to be observed from the outside are generally divided two categories,i.e., medical and industrial.

In fiberoptic endoscopes for medical use, upon inserting an fiberoptic endoscope into a human body, a fluoroscopic observation is often taken to locate accurately an inserted position of the top thereof relative to a region within the human body so as to ensure the safety of the person under examination. A certain fiberoptic endoscope for medical use, for instance a duodenum endoscope, can be utilized for the purpose of endoscopic retrograde cholangiopancreatography (ERCP) examinations wherein a contrast medium is, for a fluoroscopic observation, injected into the pancreatic and bile ducts through a tube which is inserted in a therapeutic instrument guide channel of a fiberoptic endoscope. As described above fiberoptic endoscopes for medical use have many uses in connection with fluoroscopic observation. It is said that fiberoptic endoscopes are expected to be used in connection with fluoroscopic observation more often in the future.

Consequently an optical fiber bundle in a fiberoptic endoscope in connection with fluoroscopic observation is frequently exposed to irradiation through a protective rubber tube which is a part of the fiberoptic endoscope, and thus induces coloration thereof and decreases its light transmittance. A fiberoptic endoscope having an image-tranmitting fiber bundle with such irradiation-induced coloration may be unacceptable for observing or examining an image therethrough and so may be returned to the manufacturer to replace the image-transmitting fiber bundle. But an image-transmitting fiber bundle is very expensive; and moreover the replacement thereof is extremely complex and hence very costly.

We obtained empirically the result that the fading of discolouration of the image-transmitting fiber bundle induced by irradiation can be caused by visible light radiation, and then the light transmittance thereof is restored to a degree acceptable for performing observations and examinations as described in detail in our European Patent Application No. 82300813.1.

The attachment may be at the proximate end of the endoscope probe which may be detachably connected during normal use to the light source, e.g. using a socket type connection in those circumstances the attachment may be interposable between the probe and the light source to connect the image transmitting bundle, rather than the light transmitting bundle.

According to the present invention, there is provided a fibre-optic endoscope comprising a light source, a probe comprising a light transmitting fibre-optic bundle for conducting light from the source to an area to be viewed and an image transmitting fibre bundle for conducting light picked up from the area to be received to an ocular arrangement, the image transmitting fibre bundle being susceptible to a reduction in light transmittance by irradiation, which transmittance may be restored by exposing the bundle to visible light radiation, the image transmitting fibre in normal use receiving light from the light source reflected from the area being viewed, characterised by an attachment temporarily attachable to the probe for directing light direct from the light source to the image transmitting fibre.

In another arrangement, the attachment may be

- 4 -

at the distal end of the probe and be adapted to direct
light from the light transmitting bundle to the image
transmitting bundle.

The invention will be further described by way
of example with reference to:

Figure 1 is a schematic view of a fiberoptic
endoscope for medical use;

Figure 2 is a cross sectional view of an ocular
portion of the fiberoptic endoscope of Figure 1;

Figure 3 is a cross sectional view of an interconnecting device for use in an embodiment of the present invention and which is adapted to interconnect a light source unit and an ocular portion of fiberoptic endoscope;

Figure 4 is a cross sectional view of an interconnecting device of another embodiment of the present invention which is adapted to interconnect a light source unit and one end of an image-transmitting fiber bundle through an ocular portion of a fiberoptic endoscope;

Figure 5 is a cross sectional view showing another embodiment of the present invention in which a light source is specially provided ;

Figures 6 to 9 are cross sectional views of still other embodiments of the invention which are adapted to reflect emitted light from a light-transmitting fiber bundle so as to turn the light path backward and toward the incident end of an image-transmitting fiber bundle ;

Figure 10 is a schematic view of another embodiment of the present invention wherein the distal end and the occular part of fiberoptic endoscope are optically coupled by a connector so as to expose the image-transmitting fiber bundle at its front end to visible light radiation emitted from the light-transmitting fiber bundle through an ocular assembly adjacent the fiber bundle at the front end ;

Figures 11 and 12 are cross sectional views showing embodiments of the connector shown in Figure 10 ;

Figure 13 is a schematic view of another embodiment of the present invention wherein a yellow-colored image-transmitting fiber bundle is exposed to visible light radiation through an ocular assembly when the fiberoptic endoscope is returned to its storage location ;

Figure 14 is a perspective view of a suspension means shown in Figure 13 ;

Figure 15 is a block diagram showing a control circuit for a light source ; and

Figure 16 is a partly cross sectional view of the embodiment which resemble the embodiment shown in Figure 13 wherein the location of a suspension means is adjustable.

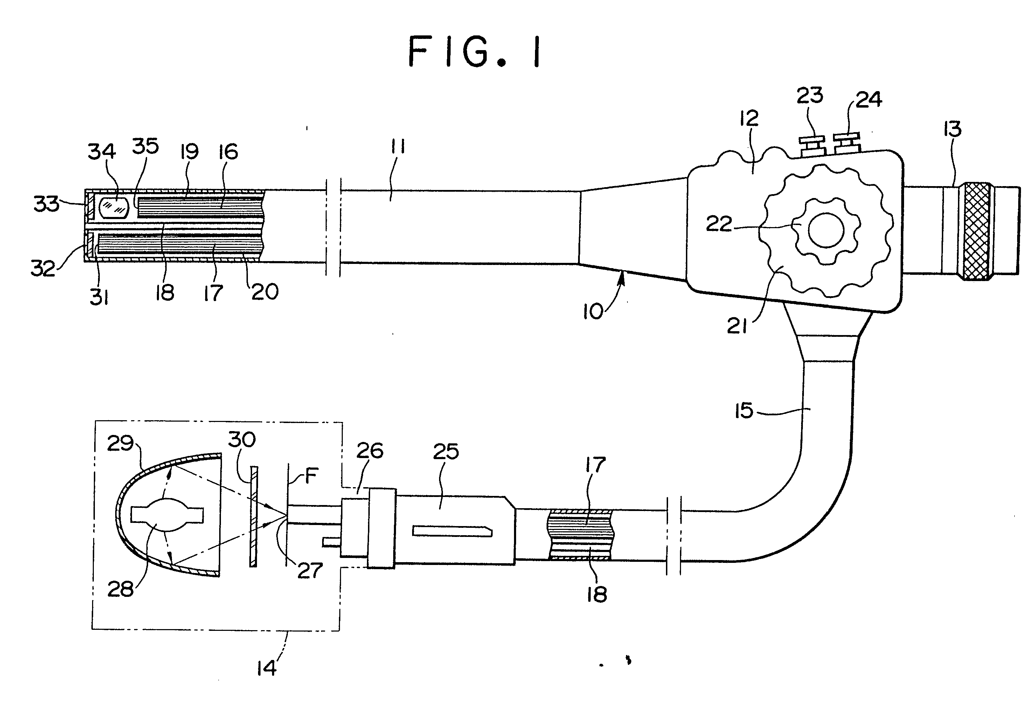

Referring now in detail to the drawings a fiberoptic endoscope 10 for medical use shown in Fig. 1 comprises an elongated flexible and tubular part 11 insertable into body cavities to be examined, a remote control 12, an ocular part 13 and an extention part 15 which is adapted to be detachably connected to an illumination light source unit 14. The elongated flexible part 11 accomodates an image-transmitting optical fiber bundle 16 and a light-transmitting optical fiber bundle 17, both of which comprise an extremely large number of optical fibers each of diameter about 10 to 20 μm, and an elongated flexible tube 18 for allowing water and air being fed into a body cavity therethrough. The optical fibers of the image-transmitting fiber bundle 16 are rigidly secured together by an adhesive such as epoxy resin so as to maintain their spatial relationship ; but they are free along their extent between their ends. On the other hand, the light-transmitting fiber bundle 17 is constructed in the same manner as the image-transmitting fiber bundle 16, but it is not required that its fibers maintain their spatial relationship at both ends. The fiber bundles 16 and 17 are covered for protection by rubber tubes 19 and 20 respectively. The elongated flexible part 11 is, as well known , adapted to be moved so as to look in any direction desired, by operating control knobs 21 and 22 of the remote control part 12. Operations of an air feed button 23 and a water feed button 24 cause an electromagnetic valve to act so as to control their flow through the tube 18.

The extention part 15 in which a fiber bundle and a tube each forming parts of the light-transmitting fiber bundle 17 and the air-water feeding tube 18, respectively, extend is provided-with a connecting plug 25. When the plug 25 is connected to a socket 26 provided on the illumination light unit 14, the incident end 27 of the light-transmitting fiber bundle 17 is located in a focal surface F where visible light emitted from a light source 28 is converged by a reflecting mirror 29, for instance parabolic mirror. A xenon lamp, a halogen lamp, a metal halogen or the like may be desirably employed as a light source 28. Part of the visible light emitted from the light source 28 is reflected by the mirror 29 to be directed forwardly to a heat absorbing filter 30. Light passed through the heat absorbing filter 30 falls upon the incident end 27 of the light-transmitting fiber bundle 17 and is then transmitted therethrough to an exit end 31 which is located inside of the distal end portion of the elongated flexible part 11. This light then passes through a window 32 and illuminates the field to be examined or observed. Reflected light from the observed field falls upon the image-transmitting fiber bundle 16 at the incident end 35 through a window 33 and an objective assembly 34 for forming an image thereon. The image on an exit end 36 of the image-transmitting fiber bundle 16, which has been transmitted therethrough, can be observed through an ocular assembly 37 after magbification.

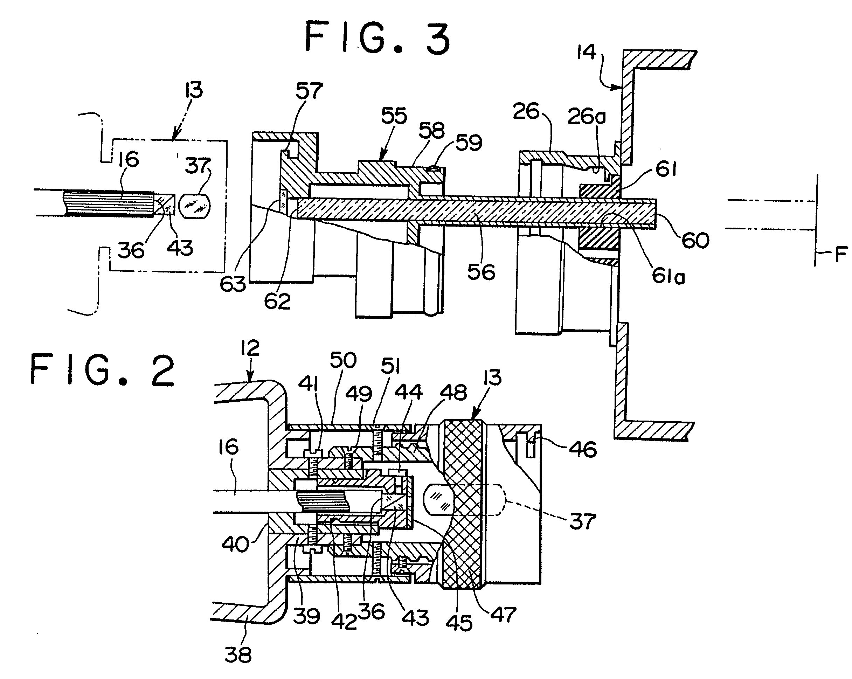

Fig. 2 shows the ocular parts construction, wherein a body portion 38 of the remote control part 12 is integrally provided with a cylindrical projection 39 into which a cylindrical ring 40 is fitted and secured thereto by set screws 41. The cylindrical ring 40 at its bottom has a center bore through which the image-transmitting fiber bundle 16 extends. A prism holder 42 which is fitted into the cylindrical ring 40 accomodates therein a half prism 43 provided with a half mirror at an angle of 45$^\circ$, a light receiving element 44 for receiving visible light reflected upwardly by the half prism, and a masking member 45. The light receiving element 44 serves to control the period of time during which the light source 28 of the light source unit 14 is energized to emit visible light when taking a photograph of a field under examination by a camera mounted on the ocular part 13 through engagement with bayonet lugs 46.

The ocular part 13 is provided with an operating ring 47 for adjusting the diopter of an ocular assembly 37 and a fixed ring 48 which is retained by set screws 49 on the cylindrical projection 39. Around a connected portion between the remote control part 12 and the ocular part 13 two semicircular cover members 50 for ornament and protection are disposed arround and secured to the fixed ring 48 by set screw 51.

Upon inserting a fiberoptic endoscope into a human body for observing therein, a fluoroscopic observation is often taken to locate accurately an insert position of the top thereof. Consequently, fiberoptic endoscopes for medical use have many uses in connection with fluoroscopic observation. As a result, the optical fiber bundle is frequently exposed to irradiation through a protective rubber tube ; the fibers of the bundle become discolored and there is thus a reduction in light transmittance. This makes observation or examination difficult or impossible. The irradiation-induced reduction in light transmittance of the image transmitting fiber bundle is restored to a degree accepatble for performing observations and examinations by visible light radiation endwise therethrough. Visible light of short wavelength and high radiation density is significantly more effective to restore light transmittance in a short time.

Fig. 3 shows part of an embodiment of the present invention wherein a light unit is connected to the ocular part of a fiber optic endoscope through a connector device. A connector device 55 is provided with a light transmitting member 56 extending therein, a first connector means at one end and a second connector means at the opposite end thereof. The first connector means is comprised by a bayonet mount having bayonet lugs 57 engageable with complementary bayonet lugs 46 of the ocular part 13 and so adapted to detachably connect the connector device 55 to the ocular part 13. The second connector means is comprised by a cylindrical sleeve 58 with an outer diameter smoothly fittable into a socket 26 of the light source unit 14 and an annular click spring 59 engageable with an annular groove 26 formed on the inner surface of the socket 26, and thereby detachably connects the connector device 55 to the light source unit 14. Employed as the light transmitting member 56 may be a transparent glass rod, optical glass fiber bundle or the like which is supported in the connector device 55 in such a way that the light transmitting member 56 extends beyond the foremost of the cylindrical sleeve 58 and the incident end 60 thereof is, when the interconnection between the second connector means and the socket 26 is completed, located in the focal surface F where visible light emitted from the light source 28 (see Fig.1) is converged. Further the light transmitting member 56 at its exit end 62 faces the ocular assembly 37 when the interconnection between the first connector means and ocular part 13 is completed. As shown in Fig. 3, the connector device 55 is provided with a heat absorbing filter for isolating the radiant heat contained in visible light emitted from the light source 28. Although the heat absorbing filter 63 is, in this embodiment, located adjacent the exit end of the light transmitting member 56, it may be located adjacent the incident end thereof, in the light source unit 14 or the ocular part 13 of the fiberoptic endoscope.

Upon using the connector device 55 constructed as described above, the connector device 55 at the second connector means is, after it has been connected to the ocular part 13 through its bayonet lugs of the first connector means, inserted into the socket 26 with the light transmitting member 56 being passed through a bore 61a surrounded by a rubber ring 61 provided within the socket 26 until the annular click spring 59 is sufficiently engaged in the annular groove 26a. The interconnection between the fiberoptic endoscope 10 and the light source device 14b by the connector device 55 causes the light transmitting member 56 to be located in position so that the incident end 60 thereof coincides with the focal surface $\underline{F}$ and the opposite thereof optically faces the ocular assembly 37. Thus the light transmitting member 56 extending between the light source 28 and the ocular assembly can expose the image-transmitting fiber bundle 16 at its incident end to visible light emitted from the light source 28 through the ocular assembly 37 and the half prism 43. Consequently, by the visible light irradiation, the fading of the irradiation-induced discoloration of the image-transmitting fiber bundle is caused, and thereby the irradiation-induced reduction in light transmittance thereof is restored.

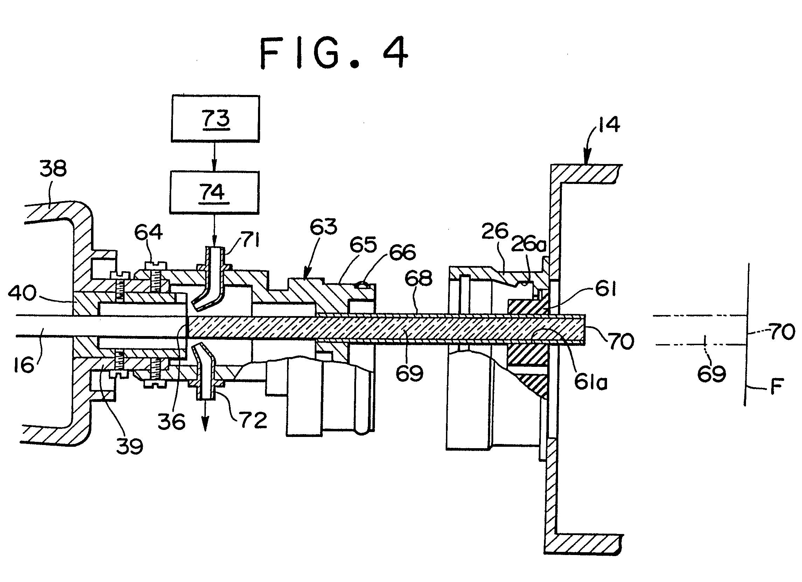

Fig. 4 shows another embodiment of the present invention in which a connector device at one end is connected to the ocular part with the ocular assembly and the half prism removed therefrom so that the light transmitting member at one end directly faces the end of the image-transmitting fiber bundle. In this case, increased visible light radiation is available because of the fact that absorption of visible light radiation by the ocular assembly and the half prism and reduction in the amount of visible light induced by the mask can be eleminated. Furthermore, since the connector is so constructed as to blow cooling air from a cooling air supply connected to the fiberoptic endoscope against the image-transmitting fiber bundle arround the one end, the adhesive such as epoxy resin by which the optical fiber at both ends are rigidly secured is prevented from blackening or being destroyed by heat. As a result, visible light of higher radiation density can be used and thus a light transmittance nearly that of the original can be regained in a short time. The connector device 63 at its forward end if fitted onto end fixed by set screws 64 to the cylindrical projection 39. And the connector device 63 at its rear end is adapted to fit into the socket 26 with the annular click spring 66 provided in the rear end engaged in the annular groove 26a formed on the inner wall of the socket 26. Further, the connector device 63 is provided with a light transmitting member 63 extending therethrough, partially sheathed by a metal tube 68. The light transmitting member 69 at its incident end 70 is located to coincide in position with the focal surface F of the reflecting mirror 29 and to be, at the
opposite or exit end positioned adjacent or in contact
with the end 36 of the image-transmitting fiber bundle
16. Provided on the connector device 63 near the
boundary between the image-transmitting fiber bundle
16 and the light transmitting member 69 is an air nozzle
71 and an exhaust pipe 72. Air sent from an air blower
73 is, after cooled by a cooling device 74, supplied
into the inside of the connector device 63 through the
air nozzle 71 for cooling the boundary therebetween.
Although the cooling device 74 is, in this embodiment,
employed for cooling the air from the blower 73, it is
not necessarily to provide the cooling device 74 since
air in itself has a certain measure of cooling effect.
Further, it is desirable to employ an exhaust blower to
be connected to the exhaust pipe 72 for compulsory
exhaust. The connector device 63 is completely connected
to the socket 26 by inserting , after the insertion of
the light transmitting member 69 into the bore 61a,
the second connector means 65 thereinto till the engagement between the annular click spring 66 and the annular
groove 26a is attained. The connector device 63
completely connected to the socket 26 causes the light
transmitting member 69 at the incident end 70 to be
located in the focal surface F shown by a double dotted
chain line where visible light emitted from the light
source 28 is converged by reflecting mirror. When the
connector device 63 at its first or front end is fixed
to the cylindrical projection 30 of the remote control part 38 by set screws 64, the light transmitting member 69 at the opposite end, namely the exit end, is located to contact with the end of the image-transmitting fiber bundle. Thus, the image-transmitting fiber bundle 16 is exposed to visible light with a high radiation density through the light transmitting member 69, causing the fading of the colored image-transmitting fiber bundle induced by irradiation and thereby recovering the light transmittance thereof to a degree acceptable for performing observations and examinations. During the exposing of visible light to the image-transmitting fiber bundle, the image-transmitting fiber bundle 16 arround its end 36 is cooled by the air blown through the air nozzle 71 which will be forced to be exhaust through the exhaust pipe 72. As a result, the blackening of epoxy resin by heat rays can be prevented, and thus visible light of high radiation density is allowed to be radiated so as to regain a light transmittance near to the original in a short time.

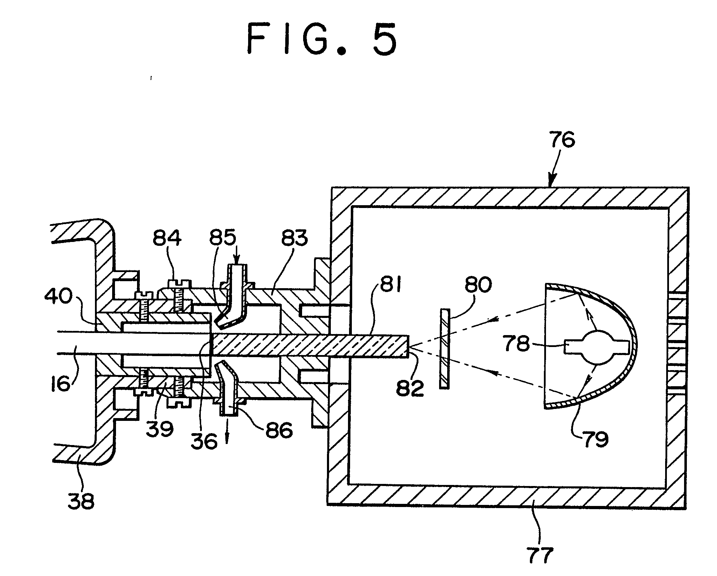

Fig. 5 shows another embodiment of the present invention in which a specially-provided light source is used for causing the fading of a colored image-transmitting fiber bundle. In this embodiment, an apparatus 76 for restoring the light transmittance of an image-transmitting fiber bundle includes a casing 77 in which a light source 78 emitting visible light containing short wavelength components such as a xenon lamp, a halogen lamp, a metal halogen lamp or the like is provided. Visible light reflected by a reflecting mirror 79 with a parabolic surface is converged at an
incident end 82 of a light transmitting member 81 through
a heat absorbing filter 80. Provided on a front wall
of the casing 77 is a cylindrical connector means 83
which is adapted to be fittable onto the cylindrical
projection 39. When the connector means 83 is fitted,
it is fixed by set screws 84 for preventing from coming
out of the projection 39. The connector means 83 is
provided with the light transmitting member 81 extending
therein the exit end of which is located adjacent or
in contact with the end 36 of the image-transmitting
fiber bundle 16. In Fig. 5, numeral 85 is an air nozzle
and numeral 86 is an air exhaust pipe. The connector
means 83 in this embodiment is the same in structure
and function as the connector device 63 shown in Fig. 4,
except that the former is integrally provided on the
casing 77.

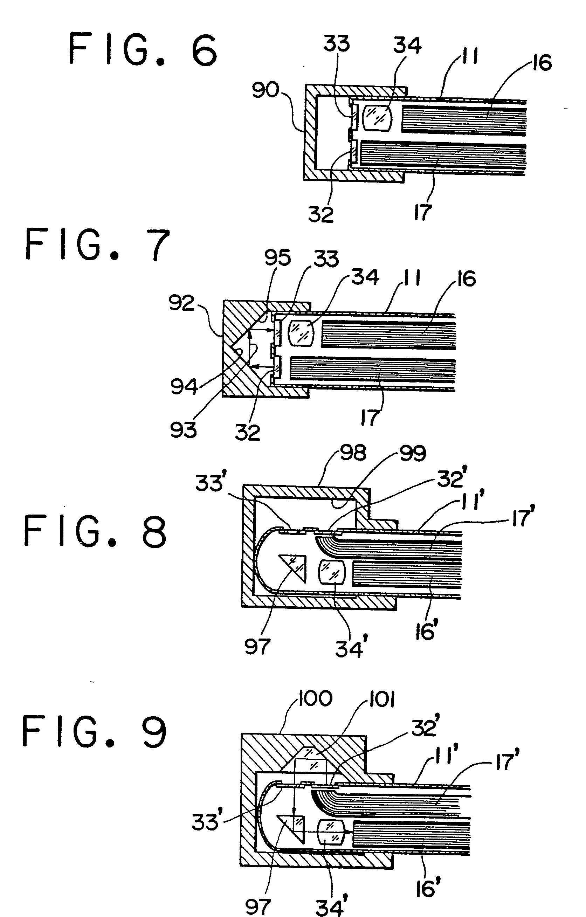

Fig. 6 is part of another embodiment of the present
invention in which the image-transmitting fiber bundle
16 at one end adjacent an objective assembly is exposed
to visible light from the light transmitting fiber
bundle accommodated with the fiberoptic endoscope. In
Fig. 6, referrence numeral 90 is a round-bottomed
cap means which is adapted to be detachably threaded
or detachably mounted by elastic force to the elongated
flexible part 11 at its forward end . The
cap means 90 is so formed as to make a space
between the bottom and the top end when it is threaded
or mounted to the elongated flexible part 11.

In addition to this, it is desirable that the inner surface of the bottom wall of the cap means 90 is a reflective surface, for instance a polished metal surface or a surface coated with a thin reflex film, which can reflect and thus deflect visible light from the light transmitting fiber bundle 17 toward the image-transmitting fiber bundle 16.

When restoring the light transmittance of a colored image-transmitting fiber bundle induced by irradiation by the apparatus as described above, the cap means 90 is, at first, threaded or elastically mounted to the elongated flexible part 11 at its top, and then the light source 28 shown in Fig. 1 is lit to emit visible light. The visible light emitted from the light source 28 is transmitted to and then emitted from the exit end of the light transmitting fiber bundle 17. The reflective surface of the cap means 90 reflects and thereby turns the visible light from the exit end through a window 32 so as to expose the image-transmitting fiber bundle 16 at the incident end to the visible light radiation through a window 33 and an objective assembly 34. As a result, the fading of the colored image-transmitting fiber bundle 16 induced by irradiation proceeds to restore progressively the light transmittance thereof.

Fig. 7 is part of another embodiment of the present invention which is the same in structure and function as that shown in Fig. 6, the reflection means is different. In this embodiment, a cap means 92 is provided with inclined reflective surfaces 94 and 95 for reflecting and thereby turning the visible
light from the light transmitting fiber bundle so as
to expose the image-transmitting fiber bundle 16 to
the deflected visible light radiation.  The cap means
using the inclined reflective surfaces in this embodiment
makes the exposure of image-transmitting fiber bundle
to visible light radiation more effective as compared
with that in the above mentioned embodiment shown in
Fig. 6.

Figs. 8 and 9 show parts of other embodiments
of the present invention which are adaptable to the
type of side-view fiberoptic endoscopes well known
to those skilled in the art.

Each of the fiberoptic endoscopes shown in Figs. 8
and 9 is the same in structure and function as that
previously described, except that the elongated flexible
part 11' along the side adjacent distal end of each
fiberoptic endoscope is provided with windows 32' and
33' for illumination and observation, and a right-angled
prism 97 for deflecting light path between the observation window 33' and an objective assembly 34', and
that a light transmitting fiber bundle 17' at its
light exit portion is curved to face the illumination
window 32'.  A cap means 98 is so formed as to surround
the window 32' and 33' therein and as to make a suitable
space between the side wall 99 and the windows 32' and
33' which the cap means 98 has been completely connected
to the top of the elongated flexible part 11'.  Further,
the inner surface of the side wall is adapted to reflect

- 18 -

light ; for instance it is coated with a light reflective film.

In Fig. 9 a cap means 100 is provided with a right-angled prism 101 for reflecting and thus turning the path of visible light from the light transmitting fiber bundle 17' toward the image-transmitting fiber bundle 16' when the cap means 100 has been completely connected to the top of the elongated flexible part 11'.

Such cap means 98 or 100 can serve, when it has been connected to the top of the elongated flexible part 11', to expose the image-transmitting fiber bundle 16' to visible light radiation of the light source 28 through the light transmitting fiber bundle, the illumination window 32', the reflecting and deflecting means 99 or 101, the observation window 33', the right-angled prism 97 and the objective assembly 34'.

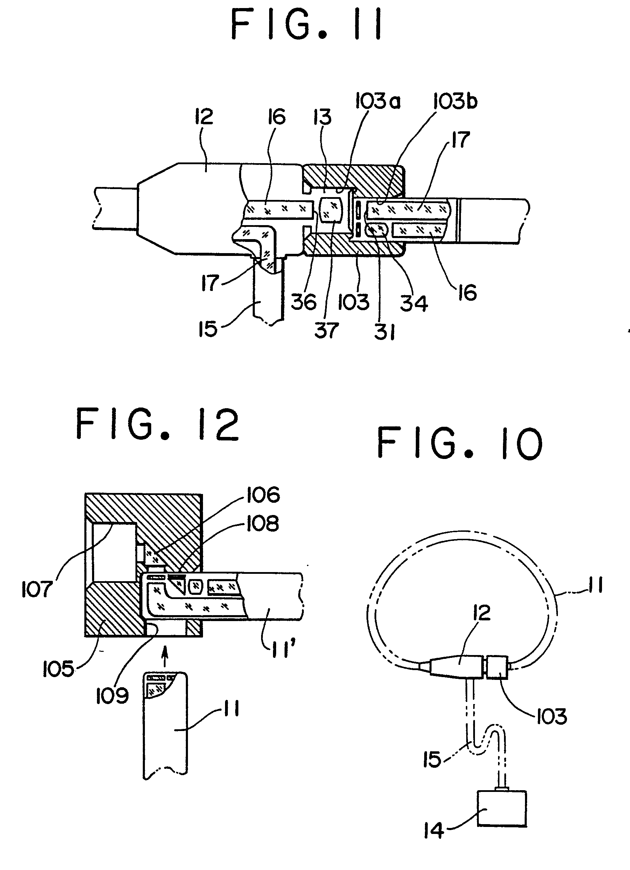

Fig. 10 shows further another embodiment of the present invention wherein an image-transmitting fiber is exposed to visible light radiation from a light transmitting fiber bundle through an ocular part of fiberoptic endoscope. In this embodiment, a connector device 103 is adapted to interconnect the top end of elongated flexible part 11 and the ocular part 13 so that the exit end 31 of the light transmitting fiber bundle 17 and the end adjacent the ocular assembly 37 of the image-transmitting fiber bundle 16 are optically coupled each other. In a fiberoptic endoscope with the elongated flexible part looped by the connector device 103 in a way as described just above, the image-transmitting fiber bundle 16 at its end adjacent the ocular assembly 37 is exposed to visible light radiation which is transmitted through the light transmitting fiber bundle 17 from the light source 28 in the light source unit 14.

The connector device 103 which is shown as an example in Fig. 11 is formed with bores 103a and 103b with different diameters for receiving the ocular part 13 and the top of the elongated flexible part 11, respectively, so as to optically couple the ends 36 and 31 of the image-transmitting and light transmitting fiber bundles 16 and 17 through the ocular assembly 37 and the illumination window 32. It is desirable to make the connector device 103 of elastic body, for instance elastic rubber or the like, which is frictionally engageable with the ocular part 13 and the elongated flexible part 11 so as to prevent both them from coming out during the exposure of the image-transmitting fiber bundle 16 to visible light radiation. Further, it may be also desirable to provide an optical means in cooperation with the ocular assembly for more effectively converging visible light at the incident end of the image-transmitting fiber bundle.

While the connector device described above is exclusively applied to the type of front-view fiberoptic endoscopes, the connector device may be modified to be adaptable to the type of side-view fiberoptic endoscopes as described hereinafter.

A connector device 105 shown in Fig. 12 is so formed as to be adaptable to either front
view fiberoptic endoscopes or side-view
fiberoptic endoscopes. The connector device 105 is
provided with an optical means 106, for instance a
right-angled prism, for deflecting a light path at an
angle of 90° which is located between a bore 107 receiving
the ocular part 13 and bores 108 and 109 perpendicularly
intersecting each other for receiving alternatively
the top of elongated flexible part 11' of side-view or
front-view fiberoptic endoscopes. The bore 108 for
receiving the top of elongated flexible part 11' is
parallel to the bore 107, but the other bore 109 for
receiving the top of elongated flexible part 11 is
perpendicular to the bore 108, and hence to the bore 107.

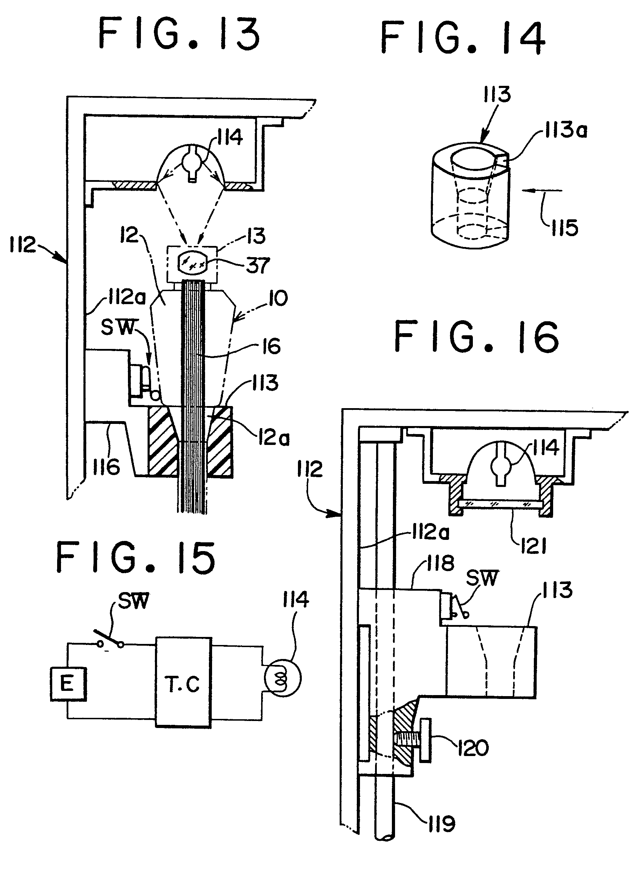

Now, fiberoptic endoscope are, when they are
unused, usually returned to a storage location, for
instance a storage locker. Thus, it is often favorable
to expose a fiberoptic endoscope with a
image-transmitting fiber bundle induced by irradiation to
visible light radiation in a storage locker with a light
source provided therein. Figs 13 to 15 show an
embodiment of such storage locker with a light source.
A storage locker 112 for resting an fiberoptic endoscope as
shown in Fig. 13 is provided with a suspension means 113
adapted to suspend the fiberoptic endoscope 10 with the
ocular part 13 positioned at a fixed location and
aligned with a predetermined direction and a light
source 114, for instance a halogen lamp, which is fixedly
positioned to align with the ocular part 13 of the fiberoptic endoscope 10 to be suspended. The suspension means 113 is made of rubber or the like and formed in cylindrical shape of a suitable hight. Along the wall of the suspension means 113 a expandable slit of a suitable width is provided for receiving the fiberoptic endoscope 10 therein. A bore surrounded by the wall is shapted to conform to the shape of the remote control part 12 at the portion 12a adjacent the elongated flexible part 11 when the fiberoptic endoscope 10 is forced into the bore with expansion of the slit 113a parallel with the arrow 115 shown in Fig. 14. The suspension means 113 is supported by means of a support bracket 116 secured to a side wall 112a of the storage locker 112. The light source 114 in the storage locker 112 is holder so as to emit visible light downwardly and to face the ocular part 13 of the fiberoptic endoscope 10 to be suspended in position by the suspension means 113. As shown in Fig. 15, the period of time during which the light source 114 is lit to emit visible light can be controlled by a circuit comprising a power source E and a timer circuit T.C. between the power source E and the light source 114. The timer circuit T.C. energized by closing a power switch SW is automatically dienergized after a duration of time previously adjusted to that required for restoring the light transmittance, and thereby the light source 114 is turned off. Consequently, the light source 114 will be automatically turned off after a predetermined length of time if the power switch SW is carelessly

- 22 -

left closed.  The power switch SW is, preferably,
a micro-switch attached to the bracket 116 and adapted
to be turned on by the remote control part 12 of the
fiberoptic endoscope 10 suspended by the suspension
means 113.

As will be apparent from the above description,
the apparatus for restoring the light transmittance of
an image-transmitting fiber bundle used in a fiberoptic
endoscope in combination with the storage locker 112
can start to expose a colored fiber bundle to visible
light radiation only by returning the fiberoptic
endoscope to its storage location in the locker after
use because the reception of the fiberoptic endoscope
in the suspension means 113 causes the power switch
SW to turn on, lightening the light source 114 to emit
visible light to which the colored image-transmitting
fiber bundle 16 should be exposed.  As a result, the
light transmittance of the reduction in light transmittance of the image-transmitting fiber bundle 16
previously exposed to irradiation can be recovered to
the degree accepatable for the following use.

Fig. 16 shows a modified embodiment of the
apparatus of Figs 13 to 15, wherein the same numerals
have been employed to denote the parts having similar
constructions and functions to those of the parts
of Figs. 13 to 15.  Therefore, the essential parts
different from those shown in Fig. 13 will be explained
hereinbelow.

In Fig. 16, referrence numeral 118 denotes a support bracket for supporting the suspension means
113 for suspending the fiberoptic endoscope in storage
location. The support bracket 118 is slidably mounted
on a guide rod 119 so as to move up and down in a
vertical line and so as to slidably contact with the
inner wall 112a of the storage locker 112 to be
prevented from rotating about the guide rod 119.
Further, the support bracket 118 is adapted to be
locked in preferable positions by means of a locking
bolt 120. Referrence numeral 121 in Fig. 16 denotes
a heat absorbing filter.

In the apparatus described just above, since
the distance between the light source 114 and the
ocular part 13 of a fiberoptic endoscope located in
the storage locker 112 can be changed by moving the
support bracket 118 up or down, the apparatus is
applicable to various type of fiberoptic endoscopes
with different distances between the ocular part and a
lower end of a remote control part so as to expose
an image-transmitting fiber bundle to sufficient
visible light radiation. In addition to this, the
radiation energy to which an image-transmitting fiber
bundle is exposed is changeable because of the fact that
the radiation density is dependent to the distance
between the source and the bundle.

Although the apparatus shown in Fig. 13 and
16 is provided with the power switch SW which is adapted
to be closed by cooperation of a fiberoptic endoscope
in the suspension means 113, the power switch SW may be

- 24 -

provided at different positions from that shown in Figs. 13 and 16 and may be manually operated. For example, the power switch SW can be provided so as to be closed in cooperation with closing operation of a front door of a storage locker. It should be further noted that the apparatus similar to that of Figs. 13 and 14 may be arranged anywhere other than storage locations in a hospital.

CLAIMS

1. An Apparatus for restoring the light transmittance of an image-transmitting fiber bundle in a fiberoptic endoscope whose light transmittance may in use be reduced by discoloration as a result of irradiation, which apparatus is adapted to be connected to said fiberoptic endoscope so as to expose said image-transmitting fiber bundle to visible light radiations, said apparatus comprising :

      a light source unit having a light source for emitting visible light radiation ; and

      a light transmitting member detachably disposed between said light source and said image-transmitting fiber bundle at one end for optically coupling them.

2. An apparatus as defined in claim 1, wherein said light transmitting member is provided in a sleeve-like connector device which has a first connector means at one end and a second connector means at the opposite end thereof, said connector device being, while coupling said light transmitting member at said one end into said light source unit, detachably connected to said light source unit through said first connector means and, while coupling said light transmitting member at the opposite end to an ocular assembly of said fiberoptic endoscope, detachably connected to the ocular part of said fiberoptic endoscope.

3. An apparatus as defined in claim 2, wherein said light source unit has a converging reflecting mirror, to converge visible light reflected thereby at a position in said unit where said light transmitting member at one end is located.

4. An apparatus as defined in claim 2 or 3 wherein said first connector means includes an annular click spring.

5. An apparatus as defined in claim 2, 3 or 4, wherein said second connector means includes bayonet lugs.

6. An apparatus as defined in any one of the preceding claims, wherein said light transmitting member is made of a glass rod.

7. An apparatus as defined in claim 1, wherein said light transmitting member is provided in a sleeve-like connector device which has a first connector means at one end and a second connector means at the opposite end thereof, said connector device being, while said light transmitting member at said one end into said light source unit, detachably connected to said light source unit through said first connector means and, while directly coupling said light transmitting member at said the opposite end to an end of said image-transmitting fiber bundle adjacent an ocular part of said fiberoptic endoscope, detachably connected to said fiberoptic endoscope with said ocular part removed.

8. An apparatus as defined in claim 7, wherein said connector device is provided with an air nozzle through which air may be circulated around said light transmitting fiber bundle at its end to be brought into contact with said light transmitting member at the said opposite end.

9. An apparatus as defined in claim 1, wherein said light source unit is provided with a connecting means on a front wall thereof which is adapted to selectively connect either a part of said fiberoptic endoscope or said light transmitting member to said light source unit.

10. An apparatus as defined in claim 1, wherein said light transmitting member comprises a light transmitting fiber bundle accommodated in said fiberoptic endoscope for transmitting visible light emitted flow said light source and a deflection means for reflecting the visible light from said light transmitting fiber bundle toward an incident end of said image-transmitting fiber bundle.

11. An apparatus as defined in claim 10, wherein said deflection means is provided with mirror surfaces which can reflect said visible light from said light transmitting fiber bundle so as to turn the path of said visible light.

12. An apparatus as defined in claim 11, said deflection means is provided on an inner wall of a cap means adapted to be coupled with a distal end of said fiberoptic endoscope.

13. An apparatus for restoring the light transmittance of an image-transmitting fiber bundle accommodated in a fiberoptic endoscope whose light transmittance may, in use, be reduced by discoloration as a result of irradiation, which apparatus is adapted to be connected to said fiberoptic endoscope so as to expose said image-transmitting fiber bundle to visible light radiation, said apparatus comprising :

      a light source unit having a light source for emitting visible light radiation ; and

      a connector device for coupling said image-transmitting fiber bundle at one end adjacent an ocular assembly with an light transmitting fiber bundle at its exit end accommodated in said fiberoptic endoscope so as to optically face each other.

14. An apparatus as defined in claim 13, wherein said connector device is adapted to loop said fiberoptic endoscope so that said image-transmitting fiber bundle at said one end and said light transmitting fiber bundle at its exit end are coupled in optically facing relation to each other.

15. An apparatus as defined in claim 14, wherein said connector device is provided with an optical means for optically confronting said image-transmitting fiber bundle at said one end said with light transmitting fiber bundle at said its exit end.

16. An apparatus for restoring the light transmittance of an image-transmitting fiber bundle accommodated as well as a light transmitting fiber in a fiberoptic endoscope whose light transmittance may in used be reduced by discoloration as a result of irradiation,which apparatus is adapted to be connected to said fiberoptic endoscope so as to expose said image-transmitting fiber bundle to visible light radiation ; said apparatus comprising:

a light source unit having a light source for emitting visible light radiation ; and

a suspension means for suspending said fiberoptic endoscope in such a way that an ocular part of said fiberoptic endoscope is located at a fixed position and in a fixed orientation so as to effectively expose said image-transmitting fiber bundle with a reduction in light transmittance induced by irradiation to visible light radiation emitted from said light source.

17.  An apparatus as defined in claim 16, said apparatus further comprising a control means for controlling a period of time during which said light source is lightned.

18.  An appartus as defined in claim 17, wherein said light source unit and said suspension means are arranged within a storage locker which is prepared for storaging fiberoptic endoscopes after used.

19.  An apparatus as defined in claim 17, said control means includes a timer means which maintain said light sorce to be energized for a predetermined period of time.

20.  A fibreoptic endoscope comprising a light source, a probe comprising a light transmitting fibreoptic bundle for conducting light from the source to an area to be viewed and an image transmitting fibre bundle for conducting light picked up from the area to be viewed to an ocular arrangement, the image transmitting fibre bundle being susceptible to a reduction in light transmittance by irradiation, which transmittance may be restored by exposing the bundle to visible light radiation, the image transmitting fibre in normal use receiving light from the light source reflected from the area being viewed, characterised by an attachment temporarily attachable to the probe for directing light direct from the light source to the image transmitting fibre.

# FIG. 1

0067555

FIG. 3

FIG. 2

# FIG. 4

# FIG. 5

FIG. 6

FIG. 7

FIG. 8

FIG. 9

0067555

# FIG. 11

# FIG. 12

# FIG. 10

# FIG. 13

# FIG. 14

# FIG. 16

# FIG. 15